# EUROPEAN PATENT APPLICATION

(11) **EP 3 673 794 A1**
(43) Date of publication of application: **01.07.2020**
(21) Application number: 19219604.6
(22) Date of filing: 24.12.2019
(51) Int. Cl.: A61B 5/00, A61B 5/042

(54) **FLEXIBLE NESTED SENSING ELECTRODES AND MANUFACTURING METHOD**

(30) Priority: 28.12.2018 US 201816235180
(71) Applicant: BIOSENSE WEBSTER (ISRAEL) LTD., 2066717 Yokneam (IL)
(72) Inventor: BEECKLER, Christopher Thomas, Irwindale, CA California 91706 (US)
(74) Representative: Small, Gary James

(57) **Abstract**

Described embodiments include an apparatus, including an intrabody probe configured for insertion into a body of a subject, and at least one electrode unit coupled to a distal portion of the probe. The electrode unit includes a flexible electrically-insulative substrate, comprising an outer surface and an inner surface, and one or more pairs of electrically-conductive coatings that coat the outer surface and are configured to sense electrophysiological signals from tissue within the body of the subject, each of the pairs including an inner coating and an outer coating that surrounds the inner coating without contacting the inner coating. The electrode unit further includes respective traces that coat the inner surface and are configured to carry the signals sensed by the electrically-conductive coatings, and multiple electrically-conductive vias that pass through the substrate and connect the traces to the electrically-conductive coatings, respectively. Other embodiments are also described.

## Description

### FIELD OF THE INVENTION

The present invention relates to intrabody probes, particularly to probes used for sensing electrophysiological signals from internal tissue of a subject.

### BACKGROUND

US Patent 6,922,579 describes a catheter tip for mapping and/or ablation. The medical probe tip includes a first ring electrode and a second ring electrode that is electrically insulated from the first ring electrode. The second ring electrode is located substantially concentric to the first ring electrode, and is coaxially surrounding the first ring electrode.

US Patent Application Publication 2017/0042615 describes a cardiac tissue ablation catheter, which includes an inflatable and flexible toroidal or spherically shaped balloon disposed at a distal region of an elongate member. The catheter further includes a flexible circuit carried by an outer surface of the balloon, the flexible circuit including a plurality of flexible branches conforming to the radially outer surface of the balloon. Each of the plurality of flexible branches includes a substrate, a conductive trace carried by the substrate, and an ablation electrode carried by the substrate, the ablation electrode being in electrical communication with the conductive trace. The catheter further includes an elongate shaft comprising a guidewire lumen extending in the elongate member and extending from a proximal region of the inflatable balloon to the distal region of the inflatable balloon and being disposed within the inflatable balloon, wherein a distal region of the elongate shaft is secured directly or indirectly to the distal region of the inflatable balloon.

### SUMMARY OF THE INVENTION

There is provided, in accordance with some embodiments of the present invention an apparatus that includes an intrabody probe, configured for insertion into a body of a subject, and at least one electrode unit coupled to a distal portion of the probe. The electrode unit includes a flexible electrically-insulative substrate, including an outer surface and an inner surface. The electrode unit further includes one or more pairs of electrically-conductive coatings that coat the outer surface and are configured to sense electrophysiological signals from tissue within the body of the subject, each of the pairs including an inner coating, and an outer coating that surrounds the inner coating without contacting the inner coating. The electrode unit further includes respective traces that coat the inner surface and are configured to carry the signals sensed by the electrically-conductive coatings, and multiple electrically-conductive vias that pass through the substrate and connect the traces to the electrically-conductive coatings, respectively.

In some embodiments, an uncoated portion of the outer surface, having a uniform width, separates the inner coating from the outer coating.

In some embodiments, the inner coating and the outer coating include gold.

In some embodiments, the electrically-insulative substrate includes a polymer.

In some embodiments, an inner perimeter of the outer coating and an outer perimeter of the inner coating have the same shape.

In some embodiments, the inner perimeter and the outer perimeter are elliptical.

In some embodiments, the inner perimeter and the outer perimeter are circular.

In some embodiments, respective surface areas of the inner coating and the outer coating are within 10% of one another.

In some embodiments, the distal portion of the probe includes one or more splines, and the electrode unit is coupled to one of the splines.

In some embodiments, the distal portion of the probe includes a balloon, and the electrode unit is coupled to the balloon.

In some embodiments, the electrode unit further includes another electrically-conductive coating that surrounds, but does not contact, the outer coating.

In some embodiments, the substrate is shaped to define a thimble.

There is further provided, in accordance with some embodiments of the present invention, a method that includes forming a plurality of electrically-conductive traces on an inner surface of a flexible electrically-insulative substrate. The method further includes forming one or more pairs of electrically-conductive coatings on an outer surface of the substrate, each of the pairs including an inner coating, and an outer coating that surrounds the inner coating without contacting the inner coating. The method further includes connecting the coatings to the traces, respectively, and coupling the substrate to a distal portion of an intrabody probe that is configured for insertion into a body of a subject.

In some embodiments, forming the traces includes forming the traces by etching away a material from the inner surface of the substrate.

In some embodiments, the material includes copper.

In some embodiments,
the coatings include a metal, and
forming the coatings and connecting the coatings to the traces includes:
drilling a plurality of channels through the substrate, such that each of the channels runs from a respective one of the traces to the outer surface of the substrate;
adhering a mask to the outer surface of the substrate, such that the mask exposes portions of the outer surface that include respective perimeters of the channels; and
subsequently to adhering the mask, plating the substrate in a plating bath of the metal, such that the metal coats the exposed portions of the outer surface, thereby forming the coatings, and fills the channels, thereby connecting the coatings to the traces.

In some embodiments, the method further includes, prior to plating the substrate, adhering a cover to the inner surface, and coupling the substrate to the distal portion of the intrabody probe includes coupling the substrate to the distal portion of the intrabody probe by adhering the cover to the distal portion of the intrabody probe.

In some embodiments, coupling the substrate to the distal portion of the intrabody probe includes coupling the substrate to a spline belonging to the intrabody probe.

In some embodiments, coupling the substrate to the distal portion of the intrabody probe includes coupling the substrate to a balloon belonging to the intrabody probe.

There is further provided, in accordance with some embodiments of the present invention, a method that includes inserting an intrabody probe into a body of a subject, at least one electrode unit being coupled to a distal portion of the probe. The electrode unit includes a flexible electrically-insulative substrate, including an outer surface and an inner surface. The electrode unit further includes one or more pairs of electrically-conductive coatings that coat the outer surface, each of the pairs including an inner coating, and an outer coating that surrounds the inner coating without contacting the inner coating. The electrode unit further comprises respective traces that coat the inner surface, and multiple electrically-conductive vias that pass through the substrate and connect the traces to the electrically-conductive coatings, respectively. The method further includes, using the pairs of electrically-conductive coatings, sensing electrophysiological signals from tissue within the body of the subject, such that the signals are carried by the vias to the traces.

The present invention will be more fully understood from the following detailed description of embodiments thereof, taken together with the drawings, in which:

### BRIEF DESCRIPTION OF THE DRAWINGS

Fig. 1 is a schematic illustration of a system for performing an electroanatomical mapping, in accordance with some embodiments of the present invention;
Fig. 1A is a close-up view of one electrode unit in the expandable catheter shown in the inset of Fig. 1;
Fig. 2A is a schematic illustration of an electrode unit, in accordance with some embodiments of the present invention;
Fig. 2B is an exploded view of Section A-A of Fig. 2A, in accordance with some embodiments of the present invention;
Fig. 2C illustrates another embodiment with more than one pair of electrodes in the form of two pairs of electrodes;
Fig. 3 is a schematic illustration of an intrabody probe, in accordance with some embodiments of the present invention;
Fig. 4 is a schematic illustration of a tip electrode, in accordance with some embodiments of the present invention; and
Fig. 5 is a flow diagram for a method for manufacturing an electrode unit, in accordance with some embodiments of the present invention.

### DETAILED DESCRIPTION OF EMBODIMENTS

### OVERVIEW

To create an electroanatomical map of a heart, a pair of electrodes (sometimes referred to as a "bipolar electrode") at the distal end of a probe may be used to detect the propagation of electric currents over the tissue of the heart. In such applications, it is typically advantageous to have one of the electrodes nested within, or enclosed by, the other. For example, the pair of electrodes may comprise an inner, circular electrode surrounded by an outer, annular electrode. This configuration allows for the propagating wave to reach the outer electrode before reaching the inner electrode regardless of the orientation of the pair of electrodes, such that the voltage difference between the pair of electrodes does not depend (or at least depends very little) on the orientation of the pair.

A challenge, however, is that manufacturing and mounting a pair of nested electrodes may be difficult, particularly if the electrodes are to be mounted onto a curved surface. To address this challenge, in embodiments of the present invention, the electrodes are formed on a flexible electrically-insulative substrate, such as a flexible polyimide substrate. Vias, which pass through the substrate, connect the electrodes to respective metallic traces on the opposite side of the substrate, such that the traces may carry the signals acquired by the electrodes. By virtue of the flexibility of the substrate, the substrate may be easily mounted onto a curved surface of the probe, such as a curved spline. Moreover, the substrate may remain firmly attached to the probe, even if the curvature of the surface changes as the probe is used.

In some embodiments, multiple substrates, each carrying one or more pairs of nested electrodes, are mounted onto respective splines at the distal end of the probe. During the electroanatomical mapping procedure, the splines are brought into contact with the subject's tissue, such that the bioelectric signals propagating through the tissue are sensed by the electrodes. Alternatively to splines, the substrates may be mounted onto a balloon.

In other embodiments, portions of the substrate that surround, but are insulated from, the nested electrodes are plated, and the substrate is shaped to define a thimble-shaped "tip electrode," which is coupled to the distal end of the probe. The tip electrode may be used for an ablation procedure, in that electric currents may be delivered from the plated portions of the substrate into the subject's tissue. During the ablation procedure, the nested electrodes may be used to sense the level of electrical activity in the tissue, such that the efficacy of the ablation procedure may be ascertained.

Typically, to manufacture the pairs of nested electrodes, metallic traces are first formed on the inner surface of the substrate. For example, if the inner surface of the substrate is initially coated by a metal such as copper or gold, the traces may be formed by etching away most of this metal.

Next, channels, which run from the traces to the portions of the outer surface of the substrate onto which the electrodes are to be deposited, are drilled through the substrate. Subsequently, a cover is applied over the inner surface, and a mask is placed onto the outer surface of the substrate. The mask exposes the channels, along with the portions of the outer surface onto which the electrodes are to be deposited, while covering the rest of the outer surface.

Subsequently, titanium-tungsten is sputtered onto the areas exposed by the mask, including the walls of the channels. Next, gold is sputtered over the titanium-tungsten. Subsequently, the substrate is placed into a gold plating bath, such that the gold layers are thickened.

In some embodiments, the cover applied to the inner surface of the substrate is a double-sided adhesive. Following the plating of the substrate, the double-sided adhesive may be adhered to a spline, a balloon, or any other suitable portion of the probe.

In addition to providing the advantages described above, the manufacturing techniques described herein facilitate controlling the conductive surface area of the electrodes with greater precision, such that, for example, the nested electrodes may have matched conductive surface areas. (In general, matched conductive surface areas maximize the signal-to-noise ratio of the acquired signals.) In contrast, if the electrodes were to be glued onto the surface of the probe, less precision would be attainable, due to the potential for some portions of the electrodes to be covered by glue. Moreover, the techniques described herein generally facilitate attaching a larger number of electrodes to the probe.

Advantageously, each pair of nested electrodes may also be used as an ablation electrode, by connecting both the inner and outer electrode to the same terminal of an ablation signal generator.

### SYSTEM DESCRIPTION

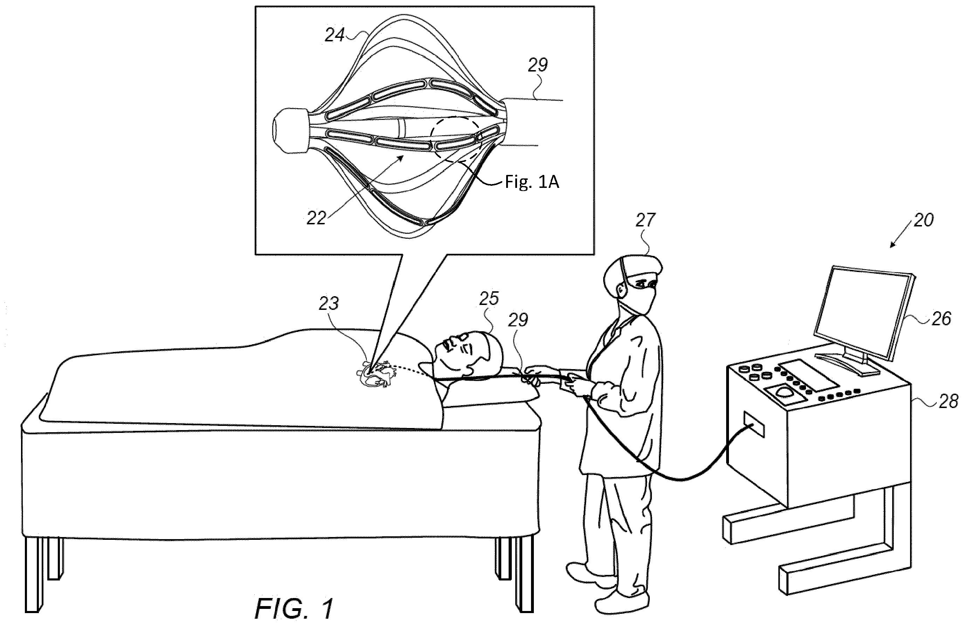

Reference is initially made to Fig. 1, which is a schematic illustration of a system 20 for performing an electroanatomical mapping, in accordance with some embodiments of the present invention.

In the electroanatomical mapping procedure depicted in Fig. 1, a physician 27 first inserts an intrabody probe 29 into the body of a subject 25. Subsequently, the physician navigates probe 29, using techniques known in the art, to a particular portion of the body of subject 25. Next, at least one electrode unit 22 coupled to the distal portion of the probe is used to sense electrophysiological signals from internal tissue of the subject. For example, the probe may be navigated to the heart 23 of the subject, and electrode unit 22 may then be used to sense electrogram signals from a myocardial surface of the subject's heart.

Probe 29 is proximally connected to a console 28, which contains a processor (not shown). As the electrode unit acquires signals from within the subject, the signals are carried, via the probe, to the processor. Based on the signals, the processor may construct an electroanatomical map for the relevant portion of the subject's body. In some embodiments, system 20 further comprises a display 26, configured to display the electroanatomical map, and/or facilitate navigation of the probe by displaying relevant images of the subject's body.

In some embodiments, the distal portion of probe 29 comprises one or more splines 24, such as a basket of splines 24 as shown in Fig. 1. (Each spline may comprise nitinol, and/or any other suitable material.) In such embodiments, at least one electrode unit 22 may be coupled to each spline. As shown in Fig. 1A, each pair of electrodes 36a and 36b for the basket catheter of Fig. 1 are in the form of flexible printed circuit with traces 40 connecting to remaining respective electrodes 36a and 36b on the end probe of Fig. 1.

### THE ELECTRODE UNIT

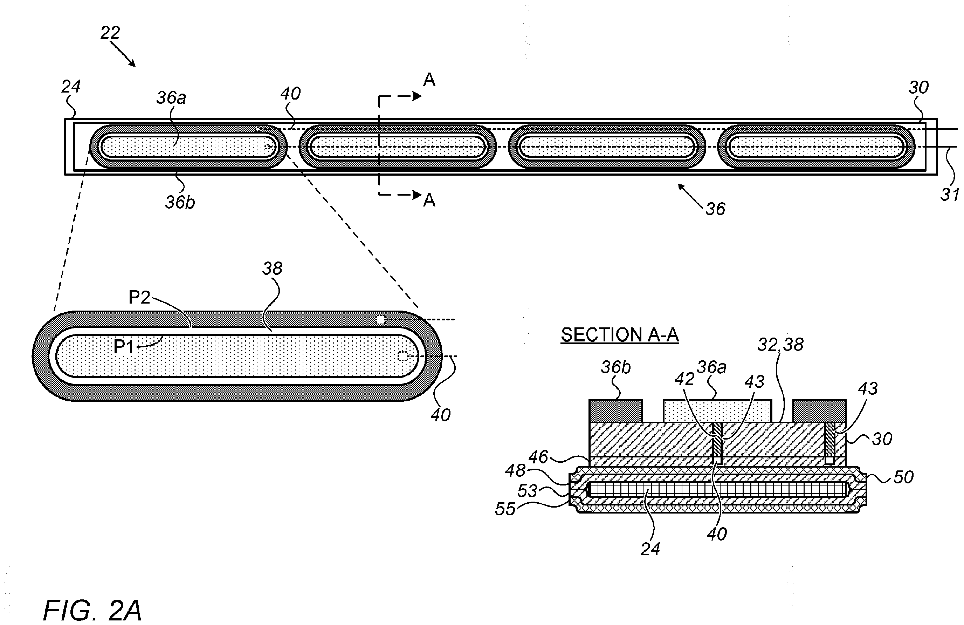

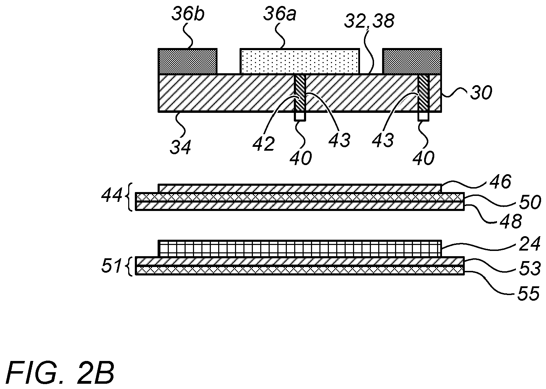

Reference is now made to Fig. 2A, which is a schematic illustration of electrode unit 22, in accordance with some embodiments of the present invention. Reference is further made to Fig. 2B, which is an exploded view of Section A-A of Fig. 2A, in accordance with some embodiments of the present invention.

Electrode unit 22 comprises a flexible electrically-insulative single-layer or multilayer substrate 30, comprising, for example, a flexible polymer, such as a polyimide. Substrate 30 comprises an outer surface 32 and an inner surface 34. One or more (e.g., between 1 and 64) pairs of electrically-conductive coatings 36 coat outer surface 32. Each pair of coatings 36 comprises an inner coating 36a and an outer coating 36b, which surrounds inner coating 36a without contacting the inner coating. In other words, outer coating 36b surrounds an uncoated portion 38 of outer surface 32, which in turn surrounds the inner coating, such that uncoated portion 38 separates the two coatings from one another. Each coating (36a or 36b) functions as a sensing electrode, by sensing electrophysiological signals as described above with reference to Fig. 1; hence, the coatings may be alternatively referred to herein as "electrodes." Coatings 36 may comprise gold, and/or any other suitable metal such as platinum or palladium.

Electrode unit 22 further comprises respective traces 40 that coat inner surface 34 and are proximally connected to respective wires 31. Multiple electrically-conductive vias 42, which pass through substrate 30, connect traces 40 to coatings 36, respectively. Each via 42 comprises a column of an electrically-conductive metal - typically, the same metal as that from which the electrodes are made - that fills a channel 43. The electrophysiological signals sensed by coatings 36 are carried by vias 42 to the traces, by the traces to wires 31, and by the wires to the proximal end of the probe. (For ease of illustration, traces 40 and wires 31 are shown for only the leftmost pair of nested electrodes.)

Typically, the outer perimeter P1 of the inner coating and the inner perimeter P2 of the outer coating have the same shape. For example, outer perimeter P1 and inner perimeter P2 may be elliptical, e.g., circular. Uncoated portion 38 is typically of uniform width, such that a gap of uniform size separates the two coatings from one another.

Typically, for improved signal-to-noise ratios, the inner and outer coatings are designed to have the same surface area. By virtue of the precise manufacturing techniques described herein, this objective may be achieved, or at least nearly achieved; for example, the respective surface areas of the inner coating and the outer coating may be within 10%, e.g., within 5%, of one another.

In some embodiments, as described above with reference to Fig. 1, electrode unit 22 is coupled to a spline 24 at the distal end of probe 29. In such embodiments, a cover 44, which covers the inner surface of the substrate, may bond the electrode unit to the spline. In some embodiments, cover 44 comprises three layers: (i) a first adhesive layer 46, which bonds to the substrate, (ii) a second adhesive layer 48, which bonds to the spline, and (iii) a middle electrically-insulative layer 50, comprising, for example, a polyimide, which is disposed between the two adhesive layers. (An example of such a cover is a Dupont™ Pyralux® LF Bond Ply.) Middle layer 50 may help insulate the spline, which is typically electrically-conductive, from traces 40. Typically, the width of middle layer 50 and second adhesive layer 48 is greater than the width of the spline (and of the substrate), such that these layers fold over, and cover, the edges of the spline.

In some embodiments, the opposite surface of the spline is covered by another insulating cover 51, comprising another electrically-insulative layer 55 that is bonded to the spline by a third adhesive layer 53. In such embodiments, the overhanging portion of second adhesive layer 48 may bond to insulating cover 51 (in particular, to third adhesive layer 53), as shown in Section A-A of Fig. 2A. Third electrically-insulative layer 55 electrically insulates the spline from the blood of the subject.

Typically, each of the adhesive layers described above comprises a partially-cured epoxy, which bonds the two relevant surfaces to one another upon being fully cured. Alternatively to using any of the adhesive layers described above, an uncured epoxy may be applied to (e.g., sprayed or rolled onto) one or both of the surfaces that are to be bonded to one another, and the uncured epoxy may then be fully cured. Alternatively, any other adhesive material may be used for the bonding.

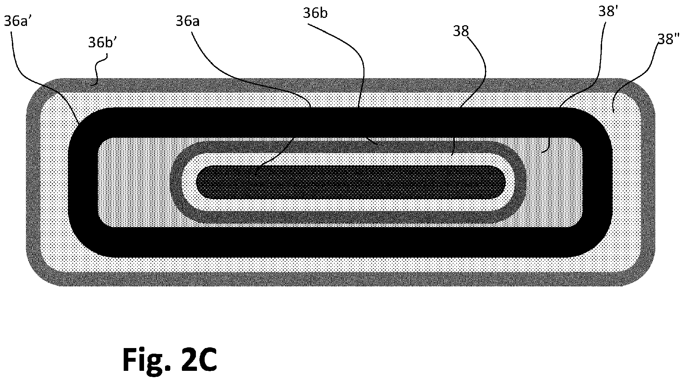

While the embodiments of Figs. 2A shows one pair of electrodes in a race track like pattern, it is within the scope of this invention to includes more than one pair of electrodes in a racetrack configuration, as shown schematically in Fig. 2C. In Fig. 2C, a first pair of electrodes includes inner electrode 36a in the form of an elongated rectangular like shape separated from first outer electrode 36b by non-conductive material 38 to prevent electrical or physical contact between electrodes 36a and 36b. A second pair of electrodes 36a' and 36b' is also provided with separation insulator 38". Note that yet a third insulator 38' is provided to separate first outer electrode 36b from second inner electrode 36a'. In this way, there are multiple pairs of electrodes depending on which electrode is being sensed. For example, a first pair of electrodes could be 36a and 36b; second pair of electrodes being 36b' and 36a'; third pair of electrodes being 36a' and 36b; fourth pair of electrodes being 36a and 36a'; fifth pair of electrodes being 36b' and 36b; and sixth pair of electrodes being 36a and 36a'.

### OTHER EMBODIMENTS

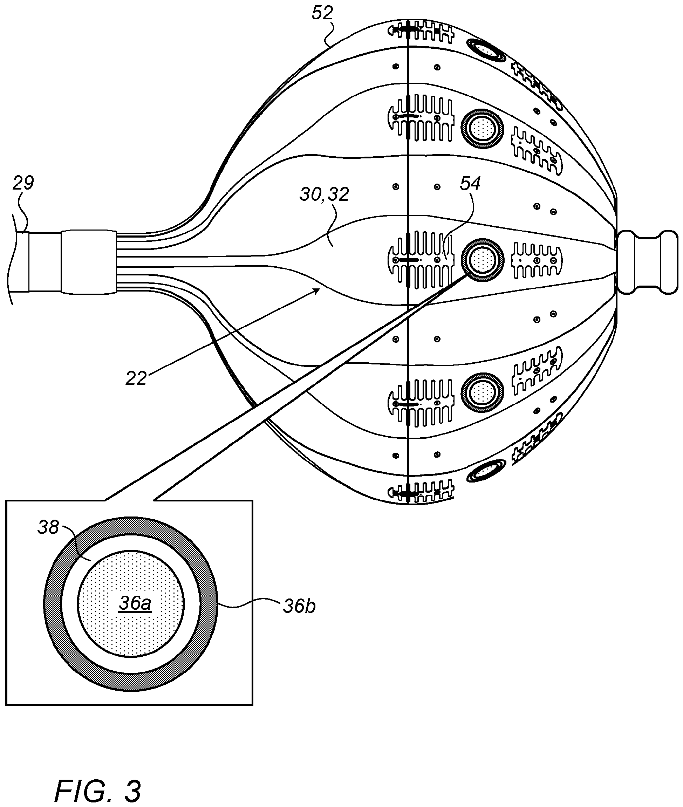

Reference is now made to Fig. 3, which is a schematic illustration of probe 29, in accordance with some embodiments of the present invention.

In some embodiments, the distal portion of probe 29 comprises a balloon 52. In such embodiments, each electrode unit 22, comprising at least one pair of nested electrodes, is coupled to the balloon. For example, as described above with reference to Figs. 2A-B, cover 44 may comprise a double-sided adhesive, which bonds to both the substrate and the balloon. (Since balloon 52 is generally not electrically-conducting, cover 44 need not necessarily comprise middle electrically-insulative layer 50.) Alternatively, cover 44 may comprise a single-sided adhesive that bonds to the substrate, and the non-adhesive surface of the cover may be bonded to the balloon by an epoxy or another adhesive material.

In some embodiments, as shown in Fig. 3, each electrode unit comprises one or more ablation electrodes 54 that coat the outer surface of substrate 30 proximally and/or distally to the nested electrodes.

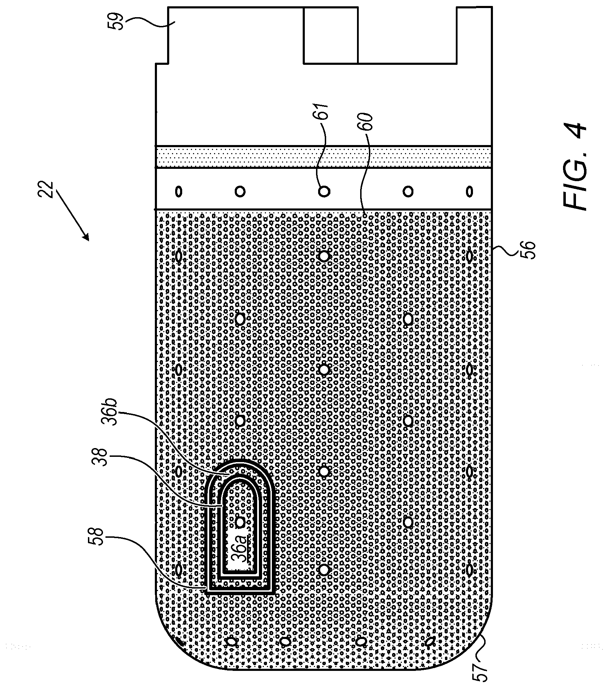

Reference is now made to Fig. 4, which is a schematic illustration of a tip electrode, in accordance with some embodiments of the present invention.

In some embodiments, electrode unit 22 further comprises another electrically-conductive coating 56 that surrounds, but does not contact (i.e., without electrical contact), the outer coating of each pair of nested electrodes. Other coating 56, which is electrically insulated from outer coating 36b by a second uncoated portion 58 of the substrate, is typically used for ablation.

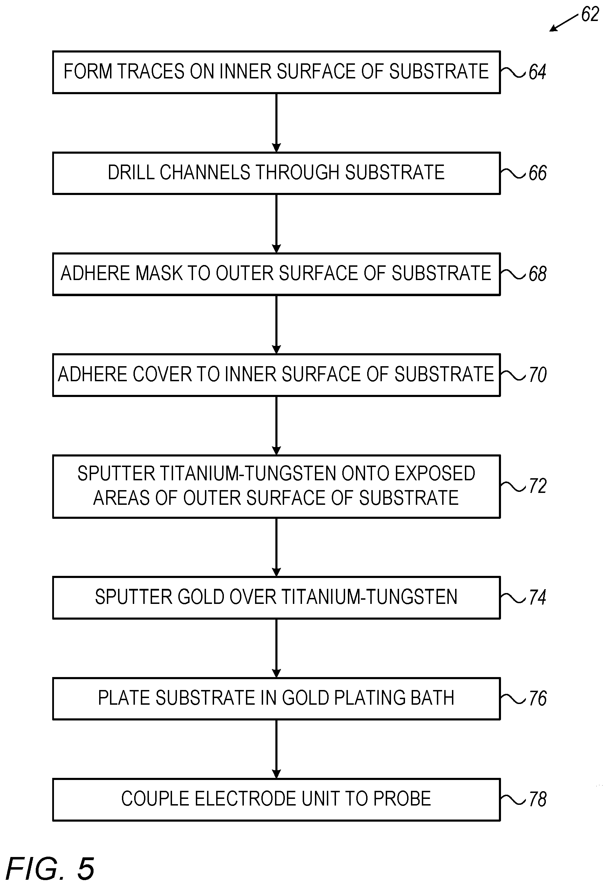

In some such embodiments, substrate 30 is deformed into a thimble-shaped tip electrode - comprising, for example, a cylindrical body capped by a dome-shaped cover 57 - that may perform both ablation and sensing functions. (An example initial shape of the substrate prior to the deformation thereof, and an example technique by which the substrate may be deformed, are described in US Application No. 16/103,806, whose disclosure is incorporated herein by reference and attached hereto in the Appendix. In particular, the initial shape of the substrate is shown in Fig. 5 of US Application No. 16/103,806, while the deformation is described with respect to Fig. 5 and to the deforming step of Fig. 4.) Following the deformation of the substrate, electrode unit 22 is coupled to the distal end of the probe, e.g., via tabs 59 at the proximal end of the substrate that fit into the main tubular body of the probe.

In such embodiments, cover 44 may comprise a single adhesive layer that adheres to the inner surface of the substrate. Typically, this adhesive layer is double-sided, and cover 44 further comprises an insulating layer bonded to the adhesive layer. This insulating layer may help insulate the traces from the interior of the tip electrode.

Typically, the substrate is shaped to define irrigation holes 61, which facilitate the outward flow of irrigating fluid during the ablation procedure. The substrate may be further shaped to define a large number of smaller holes, which, when filled with a thermally-conducting (and electrically-conducting) material, such as gold, function as thermal vias 60 for transferring heat from the ablated tissue to the irrigating fluid. Alternatively or additionally, the tip electrode may have any of the other features described in US Patent Application Nos. 15/990,532 and 16/103,806, whose disclosures are incorporated herein by reference and attached hereto in the Appendix.

### MANUFACTURING THE ELECTRODE UNIT

Reference is now made to Fig. 5, which is a flow diagram for a method 62 for manufacturing electrode unit 22, in accordance with some embodiments of the present invention.

Method 62 begins with a trace-forming step 64, at which traces 40 (Figs. 2A-B) are formed on inner surface 34 of substrate 30. For example, the inner surface of the substrate may be initially covered by an electrically-conductive material (typically a metal, such as copper), and the traces may be formed by etching away most of the material from the inner surface, leaving only the traces. Next, one or more pairs of nested electrodes are formed on outer surface 32 of the substrate, and the electrodes are connected to the traces, respectively.

Typically, the formation and connection of the nested electrodes begins at a channel-drilling step 66, at which channels 43 are drilled through the substrate, such that each of the channels runs from a respective one of the traces to the outer surface of the substrate. (The channel may pass through the trace, or may stop at the trace.) Following channel-drilling step 66, a mask is adhered to the outer surface of the substrate, at a mask-adhering step 68. The mask exposes portions of the outer surface, designated for the electrodes, which include the respective perimeters of the channels. Subsequently, the electrodes are deposited onto the exposed areas of the outer surface and the channels are filled, thus connecting the electrodes to the traces.

Typically, the electrodes are formed by a plating process, whereby the exposed portions of the outer surface of the substrate, along with the channels, are plated in a plating bath of gold. Prior to placing the substrate in the plating bath, cover 44 is adhered to the inner surface of the substrate at a cover-adhering step 70, such that cover 44 protects the traces while the substrate is inside the plating bath. (Cover 44 may also be used to couple the electrode unit to the intrabody probe, as described above with reference to Figs. 2A-B and noted below.) Additionally (either before or after cover-adhering step 70), a seed layer, comprising, for example, titanium-tungsten, is sputtered, at a first sputtering step 72, onto the exposed areas of the outer surface of the substrate (and into the channels), and gold is then sputtered, at a second sputtering step 74, over the seed layer.

Subsequently, at a plating step 76, the substrate is plated in the plating bath. In particular, the gold from the plating bath coats the portions of the outer surface exposed by the mask, thereby forming the electrodes, and fills the channels, thereby connecting the electrodes to the traces.

Subsequently, at a unit-coupling step 78, the electrode unit is coupled to probe 29 (Fig. 1), e.g., to a spline or balloon belonging to the probe. (As described above with reference to Figs. 2A-B, the electrode unit may be coupled to the probe by adhering cover 44 to the probe.) Prior to unit-coupling step 78, the substrate may be deformed into a thimble shape, e.g., as described above with reference to Fig. 4, or into any other suitable shape.

It will be appreciated by persons skilled in the art that the present invention is not limited to what has been particularly shown and described hereinabove. Rather, the scope of embodiments of the present invention includes both combinations and subcombinations of the various features described hereinabove, as well as variations and modifications thereof that are not in the prior art, which would occur to persons skilled in the art upon reading the foregoing description. Documents incorporated by reference in the present patent application are to be considered an integral part of the application except that to the extent any terms are defined in these incorporated documents in a manner that conflicts with the definitions made explicitly or implicitly in the present specification, only the definitions in the present specification should be considered.

### ASPECTS OF THE INVENTION

1. A method, comprising:
   inserting an intrabody probe into a body of a subject, at least one electrode unit being coupled to a distal portion of the probe, the electrode unit including:
      a flexible electrically-insulative substrate, including an outer surface and an inner surface,
      one or more pairs of electrically-conductive coatings that coat the outer surface, each of the pairs including an inner coating, and an outer coating that surrounds the inner coating without contacting the inner coating,
      respective traces that coat the inner surface, and
      multiple electrically-conductive vias that pass through the substrate and connect the traces to the electrically-conductive coatings, respectively; and
   using the pairs of electrically-conductive coatings, sensing electrophysiological signals from tissue within the body of the subject, such that the signals are carried by the vias to the traces.

## Claims

1. Apparatus, comprising:
an intrabody probe, configured for insertion into a body of a subject; and
at least one electrode unit coupled to a distal portion of the probe, the electrode unit comprising:
a flexible electrically-insulative substrate, comprising an outer surface and an inner surface;
one or more pairs of electrically-conductive coatings that coat the outer surface and are configured to sense electrophysiological signals from tissue within the body of the subject, each of the pairs comprising:
an inner coating; and
an outer coating that surrounds the inner coating without contacting the inner coating;
respective traces that coat the inner surface and are configured to carry the signals sensed by the electrically-conductive coatings; and
multiple electrically-conductive vias that pass through the substrate and connect the traces to the electrically-conductive coatings, respectively.

2. An electrode unit comprising:
a flexible substrate; and
at least one electrode pair that includes a first electrode defining an elongated shape and a second electrode defining an elongated perimeter enclosing the elongated first electrode without direct contact between the first electrode and the second electrode.

3. The apparatus according to claim 1, wherein an uncoated portion of the outer surface, having a uniform width, separates the inner coating from the outer coating.

4. The apparatus according to claim 1, wherein the inner coating and the outer coating comprise gold.

5. The apparatus according to claim 1, wherein the electrically-insulative substrate comprises a polymer.

6. The apparatus according to claim 1, wherein an inner perimeter of the outer coating and an outer perimeter of the inner coating have the same shape.

7. The apparatus according to claim 5, wherein the inner perimeter and the outer perimeter are elliptical.

8. The apparatus according to claim 6, wherein the inner perimeter and the outer perimeter are circular.

9. The apparatus according to claim 1, wherein respective surface areas of the inner coating and the outer coating are within 10% of one another.

10. The apparatus according to claim 1, wherein the distal portion of the probe comprises one or more splines, and wherein the electrode unit is coupled to one of the splines.

11. The apparatus according to claim 1, wherein the distal portion of the probe comprises a balloon, and wherein the electrode unit is coupled to the balloon.

12. The apparatus according to claim 1, wherein the electrode unit further comprises another electrically-conductive coating that surrounds, but does not contact, the outer coating.

13. The apparatus according to claim 12, wherein the substrate is shaped to define a thimble.

14. A method, comprising:
forming a plurality of electrically-conductive traces on an inner surface of a flexible electrically-insulative substrate;
forming one or more pairs of electrically-conductive coatings on an outer surface of the substrate, each of the pairs including:
an inner coating, and
an outer coating that surrounds the inner coating without contacting the inner coating;
connecting the coatings to the traces, respectively; and
coupling the substrate to a distal portion of an intrabody probe that is configured for insertion into a body of a subject.

15. The method according to claim 14, wherein forming the traces comprises forming the traces by etching away a material from the inner surface of the substrate.

16. The method according to claim 15, wherein the material includes copper.

17. The method according to claim 14,
wherein the coatings comprise a metal, and
wherein forming the coatings and connecting the coatings to the traces comprises:
drilling a plurality of channels through the substrate, such that each of the channels runs from a respective one of the traces to the outer surface of the substrate;
adhering a mask to the outer surface of the substrate, such that the mask exposes portions of the outer surface that include respective perimeters of the channels; and
subsequently to adhering the mask, plating the substrate in a plating bath of the metal, such that the metal coats the exposed portions of the outer surface, thereby forming the coatings, and fills the channels, thereby connecting the coatings to the traces.

18. The method according to claim 17, further comprising, prior to plating the substrate, adhering a cover to the inner surface, wherein coupling the substrate to the distal portion of the intrabody probe comprises coupling the substrate to the distal portion of the intrabody probe by adhering the cover to the distal portion of the intrabody probe.

19. The method according to claim 14, wherein coupling the substrate to the distal portion of the intrabody probe comprises coupling the substrate to a spline belonging to the intrabody probe.

20. The method according to claim 14, wherein coupling the substrate to the distal portion of the intrabody probe comprises coupling the substrate to a balloon belonging to the intrabody probe.
